# EUROPÄISCHE PATENTSCHRIFT

(11) **EP 3 659 556 B1**
(45) Veröffentlichungstag und Bekanntmachung des Hinweises auf die Patenterteilung: **21.04.2021**
(21) Anmeldenummer: 18208744.5
(22) Anmeldetag: 28.11.2018
(51) Int. Cl.: A61F 2/34, A61F 2/30, A61F 2/40

(54) **SCHALE ZUM EINSCHLAGEN IN EINE KNOCHENSUBSTANZ FÜR EINE GELENKPROTHESE**
PROSTHESIS FOR INSERTION INTO A BONE SUBSTANCE FOR A JOINT PROSTHETIC
COQUILLE DESTINÉE À ÊTRE ENFONCÉE DANS UNE SUBSTANCE OSSEUSE POUR UNE PROTHÈSE ARTICULAIRE

(43) Veröffentlichungstag der Anmeldung: 03.06.2020
(73) Patentinhaber: Jossi Holding AG, 8546 Islikon (CH)
(72) Erfinder: Gugler, Christian, 8500 Frauenfeld (CH); Jossi, Armin, 8500 Frauenfeld (CH); Schmidt, Martin, 8532 Warth (CH); Meyenhofer, Andreas, 8255 Schlattingen (CH)
(74) Vertreter: Hepp Wenger Ryffel AG

(56) Entgegenhaltungen:
- EP-A1- 1 806 112
- EP-A1- 2 502 604
- DE-A1-102006 017 473
- US-B1- 6 231 612

## Beschreibung

Die Erfindung ist in den beigefügten Ansprüchen definiert. Hier offenbarte Aspekte, Ausführungsformen und Beispiele, die nicht in den Umfang der beigefügten Ansprüche fallen, sind nicht Teil der Erfindung und werden lediglich zu Veranschaulichungszwecken bereitgestellt.

Die Erfindung betrifft eine Schale zum Einschlagen in oder auf eine Knochensubstanz für eine Gelenkprothese gemäss Anspruch 1. Derartige Schalen werden auch Einschlagpfannen genannt, welche im Gegensatz zu eingeschraubten oder einzementierten Pfannen in eine vorher ausgefräste Ausnehmung im Knochen eingeschlagen werden. Dabei ergibt sich bei einem guten Zustand der Knochensubstanz eine ausreichende Primärstabilität, bis das darauf einsetzende Knochenwachstum an der Pfannenoberfläche die Prothese zusätzlich stabilisiert.

Durch die EP 1 411 869 B1 ist eine sogenannte Pressfitpfanne zum Einschlagen bekannt geworden, bei welcher auf der Aussenseite des Pfannengrundkörpers mindestens zwei Verriegelungselemente angeordnet sind, welche jeweils einen Einschlagsteg umfassen. Dieser Einschlagsteg definiert vom distalen Steganfang bis zum proximalen Stegende mindestens eine Steigung von 85° bis 60° bezogen auf die Grundfläche, was einem Drallwinkel von 5° bis 30° entspricht. Ein Nachteil dieser bekannten Gelenkpfanne besteht darin, dass die wenigen und im Verhältnis zur Pfannengrösse relativ massiv ausgebildeten Einschlagstege noch keine optimale Primärstabilität ergeben. Ausserdem sind die Einschlagstege als Verriegelungselemente ausgebildet, was gemäss einer besonders bevorzugten Ausführungsform dadurch erreicht wird, dass die Steigung der Einschlagstege gegen das polseitige Ende kontinuierlich zunimmt. Dies führt dazu, dass die Pfannenschale nach dem Einschlagen im Hüftknochen verklemmt oder verriegelt ist, so dass ein Herausdrehen entgegen der Einschlagrichtung wirkungsvoll verhindert wird. Diese Art der Verklemmung oder Verriegelung führt auch dazu, dass beim Einschlagen der Gelenkschale das Knochenmaterial teilweise weggepflügt wird, womit eine Knochenrille geschaffen wird, welche nicht mehr vollständig vom Einschlagsteg ausgefüllt ist, was jedoch die angestrebte Verankerung wiederum schwächt.

Eine andere Oberflächenstruktur für ein Gelenkpfannenimplantat wird in der EP 2 502 604 A1 offenbart. Diese Oberfläche um-fasst auf dem Bereich zwischen dem Äquator und dem Pol der Kugelkalottenschale eine Vielzahl von Strukturelementen. Diese Strukturelemente werden durch sich überkreuzende Rillen mit entgegengesetzter Steigung gebildet. Die Rillen weisen eine Krümmung auf, so dass die Anzahl der Strukturelemente auf dem Äquator und auf einem Pol nahen Breitenkreis gleich ist.

Weitere Oberflächenstrukturen sind beispielsweise in DE 10 2006 017 473 A1, US 6 231 612 B1 oder EP 1 806 112 A1 offenbart.

Es ist daher eine Aufgabe der vorliegenden Erfindung, eine Schale der eingangs genannten Art zu schaffen, welche eine verbesserte Primärstabilität erzielt und welche möglichst schonend eingeschlagen oder aufgeschlagen werden kann, ohne dass sich im Knochenmaterial Hohlräume oder Abscherungen einstellen. Die Oberfläche der Schale sollte eine gute osteoinduktive Wirkung erzielen, womit eine Langzeitfixierung durch Osteointegration angestrebt wird. Ausserdem soll die Schale auch neuartige Anwendungsbereiche erschliessen, z.B. für schaftfreie Schulterprothesen.

Diese Aufgabe wird erfindungsgemäss mit einer Schale gelöst, welche die Merkmale im Anspruch 1 aufweist. Die Schale hat dabei eine insbesondere bezogen auf ihre Längsmittelachse im Querschnitt konvex gekrümmte, insbesondere sphärische Aussenmantelfläche oder eine konkav gekrümmte, insbesondere sphärische Innenmantelfläche, auf der eine Mehrzahl von Rippen angeordnet sind, wobei sich alle Rippen gleichsinnig unter einem vorzugsweise zunehmenden Steigungswinkel von 45° bis 85° am äquatorialen Ende zu einem polseitigen Ende auf der Aussenmantelfläche erstrecken. Der Verlauf der Rippen ist vorzugsweise so gewählt, dass jede Drehung um einen vorbestimmten Winkelwert einen konstanten Vortrieb der Pfanne bewirkt. Dieser Verlauf bewirkt, dass sich die Pfanne beim Einschlagen selbsttätig um wenige Winkelgrade in einer vorgegeben Art und Weise dreht. Unter dem Steigungswinkel wird in diesem Zusammenhang der Winkel in einer Zylinderprojektion verstanden, wobei es sich dabei analog zu Schraubengewinden um den vom Umfang und von der Schraubenlinie eingeschlossenen Winkel handelt. Demgegenüber handelt es sich bei der Steigung um die Längsverschiebung in Achsialrichtung pro Umdrehung oder pro gleichem Drehwinkel.

Jede Rippe hat eine bestimmte unterbrochene oder ununterbrochene Rippenlänge und eine bestimmte gleichbleibende oder sich verändernde Rippenhöhe sowie zwei Flankenflächen. Jede Rippe weist eine Flankenprojektionsfläche auf, die sich auf eine durch die Längserstreckung der Rippe und senkrecht zur Aussenmantelfläche bzw. zur Innenmantelfläche verlaufende Ebene bezieht. Bei dieser Flankenprojektionsfläche einer Rippe handelt es sich um die Projektionsfläche unabhängig von der Querschnittskonfiguration der Rippe und unabhängig von der Flankenneigung, jedoch unter Berücksichtigung von Unterbrüchen und Vertiefungen. Als Aussenmantelfläche oder Innenmantelfläche wird die tatsächliche Aussenfläche oder Innenfläche einer Schale vom Äquator bis zum Pol betrachtet, jedoch ohne Berücksichtigung von Montagebohrungen oder anderen Öffnungen. Die Summe der Flankenprojektionsflächen aller Rippen entspricht wenigstens einem Fünftel der ganzen Aussenmantelfläche oder der ganzen Innenmantelfläche. Die Mantelflächen können im Querschnitt in eine die Achse enthaltenen Ebene auch ellipsoid ausgebildet sein.

Durch das angegebene Verhältnis der gesamten Flankenprojektionsfläche zur Aussenmantelfläche bzw. zur Innenmantelfläche wird für alle Schalen unabhängig von deren Einsatz, Zweck oder Grösse ein optimaler Formschluss mit dem Knochen und damit auch eine optimale Primärverankerung erzielt. Ersichtlicherweise können dabei die Anzahl Rippen, die Rippenlänge und die Rippenhöhe variieren, um mindestens die gewünschte minimale kumulierte Flankenprojektionsfläche zu erreichen. Das Anbringen der Rippen auf der Innenmantelfläche und die optimierte Primärverankerung erlauben z.B. den Einsatz der Schale für schaftfreie Oberflächenersatzprothesen (bone resurfacing) insbesondere des Hüftgelenks oder des Schultergelenks.

Ersichtlicherweise stösst die Ausgestaltung und Anzahl der Rippen und damit auch die erzielbare Flankenprojektionsfläche bei den gängigen Gelenkprothesen an orthopädische und auch an fabrikationstechnische Grenzen. Besonders vorteilhaft liegt daher das Verhältnis zwischen der Summe der Projektionsfläche aller Rippen und der Aussenmantelfläche bzw. an der Innenmantelfläche im Bereich von 0,2:1 bis 1:1. Daraus ist ersichtlich, dass die Summe der Projektionsflächen ohne weiteres gleich gross sein kann wie die Aussenmantelfläche bzw. die Innenmantelfläche der eigentlichen Schale oder Kalotte.

Auf der Aussenmantelfläche bzw. der Innenmantelfläche können dabei wenigstens 20 und vorzugsweise zwischen 30 und 80 Rippen angeordnet sein. Bei dieser Anzahl Rippen ergibt sich ein ausgewogenes Verhältnis bezüglich der Dimensionierung der Rippen und deren Verteilung auf der Aussenmantelfläche oder auf der Innenmantelfläche.

Der Abstand zwischen benachbarten Rippen am äquatorialen Ende von Rippenmitte zu Rippenmitte auf der Aussenmantelfläche oder auf der Innenmantelfläche (Bogenmass) liegt vorzugsweise im Bereich von 0,2 mm bis 4 mm, besonders bevorzugt im Bereich von 1 mm bis 3,5 mm, was bei den gängigen Nenn-Schalengrössen für Hüftgelenkschalen zwischen 46 mm und 68 mm und in Abhängigkeit von der Rippenhöhe ersichtlicherweise zu einer hohen Anzahl Rippen führen kann.

Vorzugsweise beträgt die kumulierte Länge aller Rippen auch noch wenigstens 1000 mm oder mehr. Selbstverständlich schliesst diese Konfiguration der Pfanne nicht aus, dass z.B. im äquatorialen Bereich zusätzlich zu den Rippen noch einzelne zusätzliche Elemente wie Nocken oder Finnen angeordnet sind.

Idealerweise liegt das Verhältnis von Rippenabstand zu Rippenhöhe zwischen 5:1 bis 1:1. Allerdings können bei einem Rippenabstand von z.B. 0,2 mm auch nur Rippen von relativ geringer Höhe angeordnet werden. Selbstverständlich spielt dabei auch die Schalengrösse eine wesentliche Rolle. Je nach Schalengrösse, Rippenabstand und Rippenhöhe kann die kumulierte Flankenprojektionsfläche unterschiedlich ausfallen. Als Abstand zwischen benachbarten Rippen wird hier der Abstand zwischen deren Mitte an der Aussenmantelfläche oder an der Innenmantelfläche am äquatorialen Ende betrachtet. Dabei ist es denkbar, dass nicht die gesamte Aussenmantelfläche oder Innenmantelfläche bezogen auf die Breitengrade mit Rippen versehen ist. Dabei ergeben sich im rippenfreien Sektor ersichtlicherweise grössere Zwischenräume, welche nicht als Distanz zwischen benachbarten Rippen betrachtet werden.

Weitere Vorteile können erreicht werden, wenn sich wenigstens 30%, vorzugsweise wenigstens 50% der Rippen über mehr als die Hälfte der Höhe der Gelenkpfanne, bezogen auf deren Längsmittelachse, erstrecken. Ersichtlicherweise konvergieren die Rippen gegen den Polbereich, womit sich der Abstand zwischen den Rippen kontinuierlich verkleinert. Um bei gegebener Rippenhöhe trotzdem an sämtlichen Stellen einen ausreichenden Rippenabstand zu gewährleisten, ist es daher zweckmässig, wenn sich ein Teil der Rippen, typischerweise die Hälfte der Rippen, gegen den Polbereich nur bis zu einem bestimmten Breitengrad erstrecken, während die restlichen Rippen noch weiter bis in Polnähe geführt sind. Alternativ kann auch die Rippenhöhe im Verlauf der Rippen variiert werden.

Auf der Aussenmantelfläche bzw. auf der Innenmantelfläche sind vorteilhaft Rippen unterschiedlicher Länge angeordnet und zwar vorzugsweise in regelmässiger Abfolge bezogen auf den Umfang. In bestimmten Fällen wären aber auch unregelmässige Muster denkbar, wobei sich beispielsweise ein paar wenige Rippen bis in Polnähe erstrecken, während mehrere wesentlich kürzere Rippen zwischen zwei langen Rippen angeordnet sind.

Die Rippenhöhe liegt vorteilhaft im Bereich von 0,1 mm bis 4 mm, wobei diese Höhe selbstverständlich auch abhängig ist vom gewählten mittleren Abstand zwischen den Rippen und von der Schalengrösse. Dabei kann es ausserdem vorteilhaft sein, wenn auf der Aussenmantelfläche bzw. auf der Innenmantelfläche Rippen unterschiedlicher Höhe, vorzugsweise in regelmässiger Abfolge, angeordnet sind.

Weiter kann die Rippenanordnung dadurch variiert werden, dass bezogen auf den Umfang der Aussenmantelfläche bzw. der Innenmantelfläche auf einem bestimmten Breitengrad Rippen unterschiedlicher Rippenhöhe angeordnet sind.

Um eine möglichst schonende Verdrängung von Knochenmaterial zu bewirken, weisen die Rippen vorteilhaft einen sich vom Rippengrund zum Rippenscheitel verjüngenden Querschnitt, vorzugsweise einen keilförmigen Querschnitt auf. Alternativ zu einem keilförmigen Querschnitt könnte dieser auch trapezförmig oder pagodenförmig oder halblinsenförmig verlaufen. In bestimmten Fällen wäre es aber auch denkbar, dass die Rippenflanken annähernd parallel verlaufen, so dass die Rippen eine rechteckige Konfiguration erhalten. Weiter kann es vorteilhaft sein, das Querschnittsprofil der Rippen bezogen auf deren Länge kontinuierlich oder in Stufen zu variieren. Die erwähnten Querschnitte beziehen sich auf eine Ebene senkrecht zur Längserstreckung der Rippe und senkrecht zur Aussenmantelfläche bzw. zur Innenmantelfläche.

Der Verlauf der Rippensteigung wird vorteilhaft derart gewählt, dass beim Einschlagen in Einschlagrichtung sich jeder Abschnitt einer Rippe in der Rille bewegt, die von einem vorausgehenden Abschnitt gefurcht wird, so dass sich nach dem Einschlagen keine Verklemmung oder Verblockung im Knochenmaterial einstellt. Die Steigungskurve der Rippen muss dabei ersichtlicherweise die Schalenkrümmung derart berücksichtigen, dass sich ein gewindeähnlicher Verlauf ergibt, wobei die Rippen komplementäre Gewinderillen in das Knochenmaterial prägen und das Knochenmaterial dadurch die Funktion einer Gewindemutter erhält. Durch diese Massnahme wird verhindert, dass beim Einschlagen Knochenmaterial abgeschert oder weggepflügt wird, was ersichtlicherweise der Primärstabilität abträglich ist.

Die Primärstabilität nach dem Einschlagen der Gelenkschale kann weiter dadurch erhöht werden, dass die Flanken der einzelnen Rippen unterschiedliche Rauheitswerte aufweisen, derart, dass die beim Einschlagen höher belasteten Flanken den geringeren Rauheitswert aufweisen als die beim Ausziehen höher belasteten Flanken. Dadurch stellt sich eine verstärkte Selbsthemmung der Gelenkschale ein, ohne dass wie im Stand der Technik eine Verklemmung oder Verblockung stattfindet. Die unterschiedlichen Rauheitswerte bewirken lediglich eine Erhöhung der Reibung auf jeweils einer Flankenseite.

Weitere erhebliche Vorteile im Hinblick auf die Primärstabilität können erzielt werden, wenn wenigstens ein Teil der Rippen zur Bildung einzelner Rippenzähne unterbrochen sind. Eine einzelne Rippe bildet dann ersichtlicherweise eine Reihe hintereinander angeordnete Zähne. Je nach Konfiguration dieser Zähne wird dadurch das Einschlagen erleichtert und das Aushebeln oder Ausziehen erschwert. Da die Wirkung der Rippen im Äquatorbereich ersichtlicherweise anders ist als im Polbereich, ist es ausserdem zweckmässig, wenn die Rippenzähne einer Rippe zwischen dem äquatorialen Ende und dem polseitigen Ende eine unterschiedliche Konfiguration aufweisen.

Die Rippenzähne bilden vorzugsweise unregelmässige, mehrflächige Körper, mit einem viereckigen Grundriss, zwei gegeneinander geneigte Flankenseiten, mit einer dem Pol zugewandten Polseite und mit einer dem Äquator zugewandten Äquatorseite, wobei die Polseite mit der Aussenmantelfläche bzw. mit der Innenmantelfläche der Schale vorzugsweise einen kleineren Winkel einschliesst, als die Äquatorseite. Die Äquatorseite bildet damit eine Sperrfläche oder Widerhakenfläche, insbesondere wenn sie in einem Winkel von ca. 90° oder mehr auf der Aussenmantelfläche bzw. auf der Innenmantelfläche steht. Demgegenüber verhindert die Polseite mit dem flacheren Winkel ein Zerspanen des Knochenmaterials bzw. es wird ein Verdrängen des Knochenmaterials erreicht. Damit ist das Einschlagen der Schale knochenschonend und es wird eine hohe Primärstabilität erreicht.

Weitere Vorteile können erzielt werden, wenn die aufeinanderfolgenden Rippenzähne einer Rippe derart konfiguriert sind, dass in Einschlagrichtung eine Verdrängung von Knochenmaterial und in Ausdrehrichtung eine Verankerung im Knochenmaterial stattfindet. Eine derartige Wirkung kann beispielsweise durch Schränkung der Rippenzähne erreicht werden, wobei die einzelnen Rippenzähne wechselweise und vorzugsweise einseitig leicht versetzt zueinander angeordnet werden.

Um ein Eindringen der Rippen in das Knochenmaterial weiter zu erleichtern, ist es vorteilhaft, wenn wenigstens ein Teil der Rippenzähne bezogen auf den Rippenverlauf einen Querschnitt aufweist, der im polnahen Bereich dreieckig und im äquatorialen Bereich trapezförmig ausgebildet ist. Die beim Einschlagen vorausgehenden dreieckigen Rippenzähne schneiden dabei die Rillen, während die nachfolgenden trapezförmigen Rippenzähne einen möglichst optimalen Spannungsaufbau in den Rillen bewirken.

Es ist vorteilhaft, wenn sich die Zwischenräume zwischen den Rippenzähnen nicht bis zur Aussenmantelfläche bzw. bis zur Innenmantelfläche erstrecken. Das Eindringen und exakte Führen der Rippenzähne in der gefurchten Knochenrille wird verbessert, wenn ein durchgehender Rippenkörper verbleibt, auch wenn dieser selbst nur eine sehr geringe Höhe aufweist.

Zur Verbesserung der Sekundärstabilität können die Aussenmantelfläche bzw. die Innenmantelfläche und/oder die Rippen ganz oder teilweise mit einer osteoinduktiven Beschichtung versehen sein. Beschichtungen dieser Art sind im Stand der Technik an sich bereits bekannt. Dabei kann es sich beispielsweise um eine Titan-Plasma-Beschichtung oder auch um eine Hydroxyl-Apatit-Beschichtung handeln. Als Schalenmaterialien kommen Titan und Titanlegierungen in Frage, aber auch Fe- und Co-Legierungen gemäss ISO5832 oder Keramik oder Polymer-Werkstoffe. Die erfindungsgemässen Gelenkpfannen können durch Umformung, Zerspanung und/oder durch additive Herstellungsverfahren hergestellt werden.

Die erfindungsgemässe Schale kann eine Gelenkpfanne für eine Hüftgelenkprothese zum Einschlagen in ein Acetabulum sein. Alternativ kann die Schale aber auch Bestandteil einer schaftfreien Schultergelenkprothese sein, wobei die Schale einen Humerusanker zum Einschlagen in einen Humerus ist. Grundsätzlich könnte die Schale auch noch andere Verankerungsfunktionen für Prothesen am Knochenbau übernehmen. Bei einer Gelenkpfanne für eine Hüftgelenkprothese kann das Verhältnis zwischen der Summe der Flankenprojektionsfläche aller Rippen und der Aussenmantelfläche im Bereich von 0,2:1 bis 0,8:1 liegen. Demgegenüber liegt das Verhältnis bei einer Schale für einen Humerusanker eher im Bereich von 0,3:1 bis 1:1. Dieser Unterschied der Verhältnisse ergibt sich aus dem geringeren Schalendurchmesser beim Humerusanker und sich der daraus ergebenden geringeren Aussenmantelfläche.

Bei Hüftgelenkpfannen liegt die Anzahl der Rippen je nach Pfannengrösser idealerweise im Bereich zwischen 40 und 70 Rippen. Diese Anzahl kann bei Schalen für Schultergelenkprothesen allerdings unterschritten werden und im Bereich von 30 bis 40 Rippen liegen.

Ein weiterer Parameter für die erfindungsgemässe Ausgestaltung einer Schale kann das Verhältnis zwischen der Rippenhöhe und dem Durchmesser der Aussenmantelfläche bzw. der Innenmantelfläche sein. Demnach ist es besonders vorteilhaft, wenn das Verhältnis zwischen der Rippenhöhe vom Rippengrund zum Rippenscheitel und dem Durchmesser der Aussenmantelfläche bzw. der Innenmantelfläche am Äquator im Bereich von 0,001:1 bis 0,150:1, vorzugsweise im Bereich von 0,01:1 bis 0,04:1 liegt.

Weitere Einzelmerkmale und Vorteile der Erfindung ergeben sich aus den nachfolgend beschriebenen Ausführungsbeispielen und aus den Zeichnungen. Es zeigen:
- Figur 1:: Die Seitenansicht einer Gelenkpfanne in stark vergrösserter Darstellung,
- Figur 2:: eine hälftige Draufsicht auf die Gelenkpfanne gemäss Figur 1,
- Figur 3:: eine perspektivische Darstellung der Gelenkpfanne gemäss Figur 1,
- Figur 4:: eine hälftige Seitenansicht in schematischer Darstellung mit unterschiedlichen Rippenzähnen,
- Figuren 5 bis 10:: verschiedene Rillenprofile in stark vergrösserter und schematischer Darstellung,
- Figur 11:: eine Draufsicht auf gegenseitig verschränkte Rippenzähne,
- Figur 12:: eine perspektivische Darstellung eines einzelnen Rippenzahns,

- Figur 13:: ein Querschnitt durch den Rippenzahn gemäss Figur 12,
- Figur 14:: die schematische Darstellung einer Hüftgelenkprothese im Acetabulum,
- Figur 15:: die schematische Darstellung einer Schultergelenkprothese mit Humerusanker,
- Figur 16:: eine Seitenansicht auf die Breitseite eines Humerusankers mit ellipsoidem Querschnitt,
- Figur 17:: eine Seitenansicht auf die Schmalseite des Humerusankers gemäss Figur 16,
- Figur 18:: eine Draufsicht auf den Humerusanker gemäss Figur 16,
- Figur 19:: einen Querschnitt durch die Ebene I-I des Humerusankers gemäss Figur 18,
- Figur 20:: eine schematische und stark vergrösserte perspektivische Darstellung einer Rippe mit eingezeichneter Flankenprojektionsfläche, und
- Figur 21:: die schematische Darstellung einer Schultergelenkprothese als Oberflächenersatz mit ankerloser Gelenkschale.

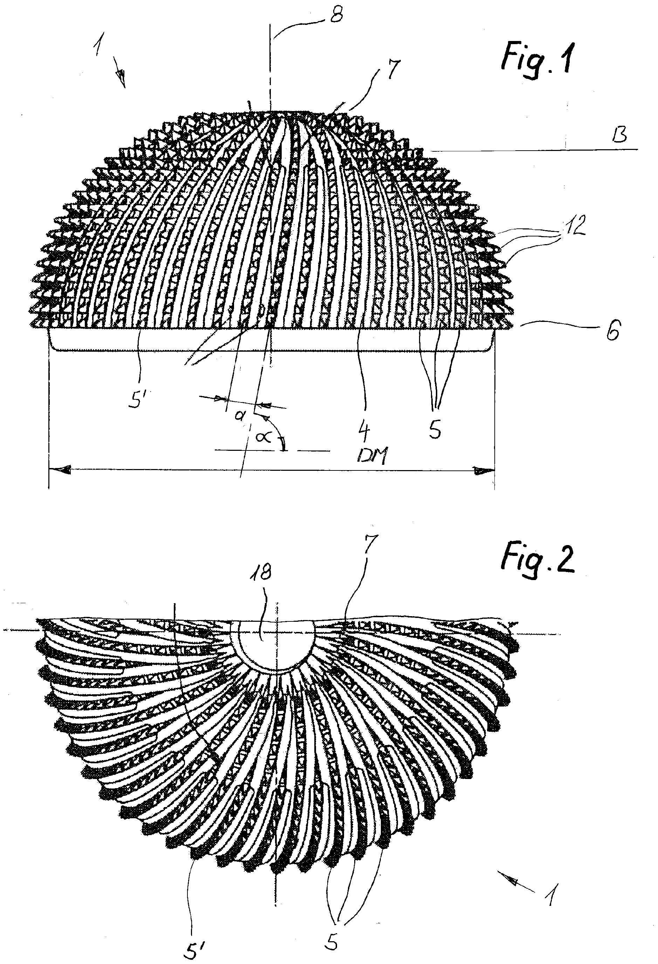

Wie in den Figuren 1 bis 3 dargestellt, besteht eine Gelenkpfanne 1 aus einem schalenförmigen Körper mit einer Aussenmantelfläche 4, auf welcher über den gesamten Umfang verteilt eine Mehrzahl von Rippen 5 angeordnet sind. Diese erstrecken sich von einem äquatorialen Ende 6 bis zu einem polseitigen Ende 7. Im Polbereich ist die Gelenkpfanne abgeflacht und auf der Längsmittelachse 8 ist eine Polöffnung 18 angeordnet. Jede einzelne Rippe besteht aus einer Mehrzahl von hintereinander angeordneten Rippenzähnen 12. Ausserdem verlaufen nicht alle Rippen bis in Polnähe. Jede zweite Rippe 5' erstreckt sich nur bis zu einem Breitengrad B, der vom Pol etwas weiter entfernt ist. Die halbkugelförmige Schale weist am Äquator einen Schalendurchmesser DM auf, der etwas grösser ist als der Durchmesser des Fräsers, mit dem die Knochensubstanz ausgefräst wird.

Die Distanz a zwischen zwei Rippen, jeweils bezogen auf die Mitte einer einzelnen Rippe am Rippengrund, ist am äquatorialen Ende am grössten und nimmt gegen das polseitige Ende ab. Wie einleitend erwähnt, beträgt diese Distanz zwischen 0,2 mm bis 4 mm. Der Steigungswinkel α liegt am Äquator im Bereich von 45° bis 85° und vergrössert sich gegen den Pol.

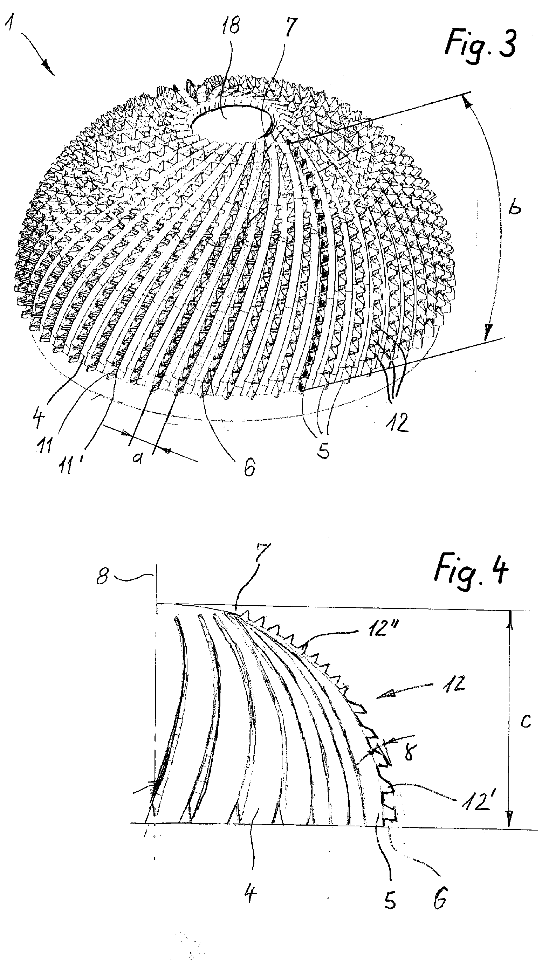

Die Länge b einer Rippe bezieht sich jeweils auf deren räumlichen Verlauf bzw. auf deren Abwicklung, wie in Figur 3 dargestellt. Die kumulierte Länge aller Rippen ergibt sich somit aus der Addition der Länge sämtlicher Rippen, unabhängig davon, ob es sich um eine Mischung von längeren und kürzeren Rippen handelt.

Jede Rippe verfügt über beidseitige Rippenflanken 11 und 11". Die Flankenprojektionsfläche P einer Rippe ergibt sich aus der Rippenlänge b multipliziert mit der Rippenhöhe d bzw. mit der gemittelten Rippenhöhe, falls diese über die Länge der Rippe variiert, abzüglich die Zwischenräume zwischen den Zähnen. Mit dieser Berechnungsart wird ersichtlicherweise die Nettoprojektionsfläche einer Rippenflanke ermittelt, ohne Berücksichtigung von deren Schräglage und Krümmung. Die Projektionsfläche ist jedoch diejenige Fläche, welche bezüglich Primärstabilität die grösste Rolle spielt. Insgesamt berechnet sich somit die kumulierte Flankenprojektionsfläche einer Pfanne aus der Anzahl Rippen multipliziert mit der Flankenprojektionsfläche einer einzelnen Rippe.

Einzelheiten eines Rippenprofils sind in Figur 4 dargestellt. Die Rippen sind dabei in einzelne Rippenzähne 12 unterteilt, wobei im äquatornahen Bereich etwa bis zur Hälfte der Gelenkpfannenhöhe c im Querschnitt trapezförmige Rippenzähne 12' angeordnet sind. Im polnahen Bereich haben die Rippenzähne 12" einen dreieckigen Querschnitt. Der Querschnitt bezieht sich dabei auf die Längserstreckung einer Rippe vom äquatornahen Ende 6 bis zum polseitigen Ende 7. Die trapezförmigen Rippenzähne 12' haben auf ihrer Scheitellinie in Bezug auf den Krümmungsradius der Rippe einen Freiwinkel γ, womit das Verdrängen von Knochenmaterial im kritischen Bereich der grösseren Durchmesser verbessert wird.

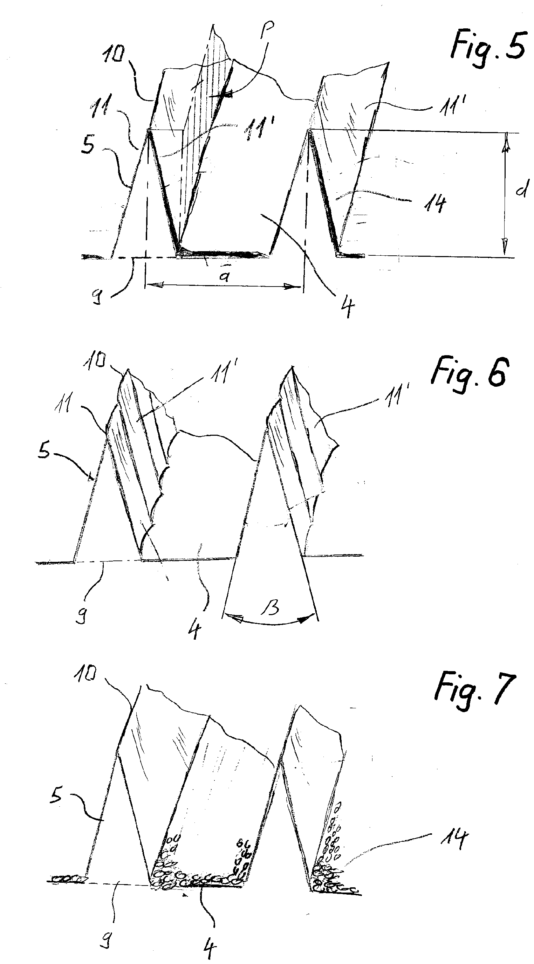

Figur 5 zeigt schematisch zwei Rippen 5 mit identischem Querschnitt mit dem Rippengrund 9 und dem Rippenscheitel 10. Die Rippenflanken 11, 11' treffen im Rippenscheitel zusammen, so dass sich im Querschnitt quer zur Längserstreckung einer Rippe eine Dreiecksform ergibt. Die Rippenhöhe d bemisst sich vom Rippengrund 9 bis zum Rippenscheitel 10. Beim hier dargestellten Ausführungsbeispiel sind die gleichsinnig geneigten Rippenflanken 11' mit einer osteoinduktiven Beschichtung 14 versehen. Diese erstreckt sich bis in den Bereich der Aussenmantelfläche 4 zwischen zwei Rippen. Der Abstand a zwischen zwei Rippen, also der Abstand von Mitte zu Mitte Rippengrund ist hier zum besseren Verständnis nochmals dargestellt. Stellvertretend für alle Ausführungsbeispiele ist hier die Flankenprojektionsfläche P einer Rippe dargestellt, die sich aus der Rippenhöhe d und der Rippenlänge b ergibt, vorausgesetzt, die Rippenhöhe bleibt gleich und die Rippe weist keine Unterbrüche auf.

Gemäss Figur 6 haben die Rippen 5 eine ähnliche Querschnittsform wie beim Ausführungsbeispiel gemäss Figur 5. Der Flankenwinkel β kann bei allen Ausführungsbeispielen im Bereich von 10° bis 90° liegen.

Der Flankenwinkel kann zwischen dem Äquatorbereich und dem polnahen Bereich sowohl konstant verlaufen als auch variieren.

An Stelle einer osteoinduktiven Beschichtung sind gemäss Figur 6 die gleichsinnig geneigten Rippenflanken 11' im Vergleich zu den gegenüberliegenden Flanken 11 mit einer höheren Oberflächenrauheit versehen, was durch die facettierte Oberfläche angedeutet ist. Der Rauheitswert Ra kann in Einschlagrichtung beispielsweise kleiner als 3µm bis 5µm und in Ausziehrichtung grösser als 30µm sein. Ersichtlicherweise kann die unterschiedliche Oberflächenrauheit mit unterschiedlichen Bearbeitungsmethoden erzielt werden wie z.B. durch einseitiges Abspanen, Bestrahlen oder Beschichten der jeweiligen Rippenflanken.

Figur 7 zeigt ein Ausführungsbeispiel, bei dem ausschliesslich die Aussenmantelfläche 4 zwischen zwei benachbarten Rippen 5 mit einer osteoinduktiven Beschichtung 14 versehen ist.

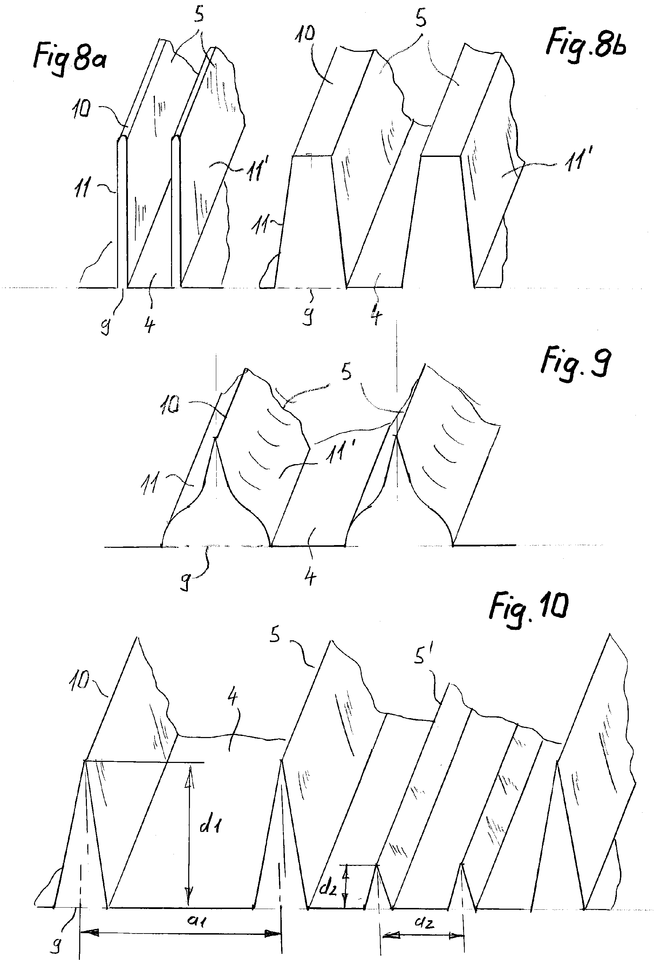

Die Figuren 8a und 8b zeigen Ausführungsbeispiele, bei denen der Rippenscheitel nicht linienförmig, sondern flach oder leicht gekrümmt verläuft. Gemäss Figur 8a verlaufen die Rippenflanken 11, 11' exakt oder annähernd parallel, so dass sich lamellenförmige bzw. im Querschnitt rechteckige Rippen 5 bilden. Demgegenüber haben die Rippen 5 gemäss Figur 8b eine im Querschnitt trapezförmige Konfiguration mit einem flachen Rippenscheitel 10.

Beim Ausführungsbeispiel gemäss Figur 9 haben die Rippen 5 einen pagodenförmigen Querschnitt mit einem recht breiten Rippengrund 9 und einem spitzwinkligen Rippenscheitel 10. Die Rippenflanken 11 und 11' verlaufen kurvenförmig.

Figur 10 zeigt schematisch ein Ausführungsbeispiel mit wechselweise angeordneten Rippen 5 und 5' von unterschiedlicher Höhe d1 und d2. Entsprechend unterschiedlich können auch die Rippenabstände a1 und a2 ausgelegt sein.

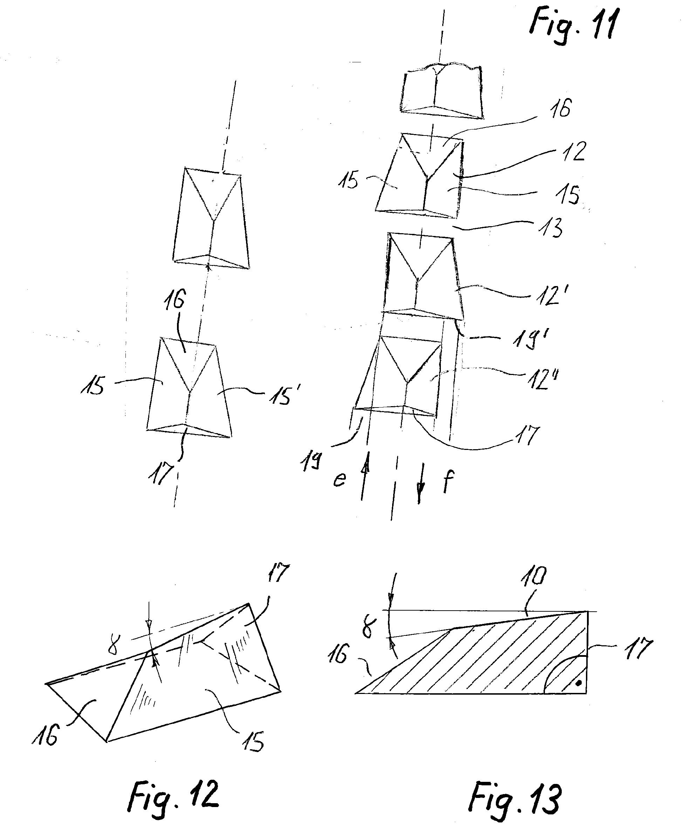

Figur 11 zeigt eine Draufsicht auf einzelne Rippenzähne 12, welche jeweils unregelmässige mehrflächige Körper darstellen, wie in den Figuren 12 und 13 ergänzend dargestellt. Die Rippenzähne der linken Reihe verlaufen regelmässig und diejenigen der rechten Reihe verschränkt zueinander. Jeder Rippenzahn 12 hat einen viereckigen, insbesondere trapezförmigen Grundriss, zwei gegeneinander geneigte Zahnflanken 15, 15', eine Polseite 16 und eine Äquatorseite 17. Die Einschlagrichtung ist mit dem Pfeil e und die Ausdrehrichtung mit dem Pfeil f dargestellt. Die Zwischenräume 13 zwischen den einzelnen Zähnen sind relativ gering. Die aufeinanderfolgenden Zähne 12' und 12" der rechten Reihe sind gegeneinander derart verschränkt, dass bezogen auf die Einschlagrichtung e der Grundriss jeweils nach rechts oder nach links auskragt. Dadurch steht einmal die Zahnflanke 15 und einmal die Zahnflanke 15' etwas weiter über die Symmetrieachse der Gesamtrille. Der Verschränkungswinkel kann zwischen 2° und 15° liegen. Daraus ergeben sich wechselweise überstehende Flanken 19, 19', welche das Lösen der Gelenkpfanne in Ausdrehrichtung f erschweren, da diese Flanken die Funktion von Widerhaken übernehmen. Dagegen wird das Einschlagen in Einschlagrichtung e durch diese Konfiguration nicht erschwert. Dazu trägt noch bei, dass die Polseite 16 mit der Grundfläche einen spitzen Winkeln einschliesst, während die Äquatorseite 17 etwa senkrecht zur Grundfläche steht.

Die nachstehende Tabelle zeigt beispielhaft die Beziehung zwischen Rippenabstand, Rippenhöhe und Anzahl Rippen, und der sich daraus ergebenden kumulierten Flankenprojektionsfläche an drei Hüftgelenk-Schalengrössen 48 mm, 52 mm und 64 mm. Als ideal wird ein Rippenabstand von 3 mm betrachtet, was eine Rippenhöhe von 1.1 mm erlaubt. Dazu sind jeweils die dabei erzielbaren kumulierten Flankenprojektionsflächen in mm2 angegeben. Die übrigen Werte sind in mm angegeben. Zusätzlich ist auch noch die Anzahl Rippen angegeben, sowie die Verhältnisse zwischen der kumulierten Flankenprojektionsflächen zur Aussenmantelfläche.

| | **min.** | **Ideal** | **max.** |
|---|---|---|---|
| Rippenabstand a | 0.2 | 3 | 4 |
| Rippenhöhe d | 0.1 | 1.1 | 4 |

| **Parameter an Schale Grösse 52 gerechnet Aussenmantelfäche: 4412 mm2** | | | |
|---|---|---|---|
| kum. Flankenprojektionsfläche | 1'416 | 1'085 | 3'270 |
| Anzahl Rippen | 834 | 56 | 42 |
| Verhältnis | 0,32 | 0,25 | 0,74 |

| **Parameter an Schale Grösse 64 gerechnet Aussenmantelfäche: 6637mm2** | | | |
|---|---|---|---|
| kum. Flankenprojektionsfläche | 2'127 | 1'651 | 4'846 |
| Anzahl Rippen | 1'022 | 70 | 52 |
| Verhältnis | 0,32 | 0,25 | 0,73 |

| **Parameter an Schale Grösse 48 gerechnet Aussenmantelfäche: 3771mm2** | | | |
|---|---|---|---|
| kum. Flankenprojektionsfläche | 1'209 | 935 | 2'909 |
| Anzahl Rippen | 770 | 52 | 40 |
| Verhältnis | 0,32 | 0,25 | 0,77 |

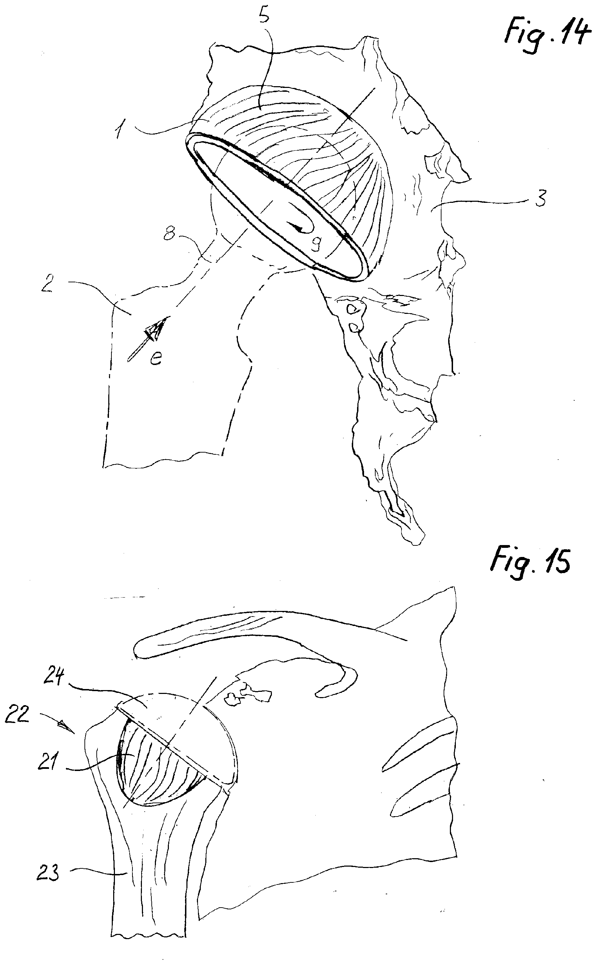

Figur 14 zeigt eine symbolische Darstellung einer Hüftgelenkprothese 2, von welcher der Schaft mit der Gelenkkugel nur andeutungsweise dargestellt ist. Die Hüftgelenkpfanne 1 mit ihren Rippen 5 ist in das Acetabulum 3 in Pfeilrichtung e eingeschlagen, wobei sie eine geringfügige Drehung in Pfeilrichtung g ausführt.

Im Gegensatz dazu zeigt Figur 15 eine symbolische Darstellung einer schaftfreien Schultergelenkprothese 22, bei der die erfindungsgemässe Schale keine Gelenkpfanne ist, sondern die Funktion eines Humerusankers 21 übernimmt, der in den Humerus 23 eingeschlagen ist. Der mit dem Humerusanker verbundene Humeruskopf 24 übernimmt dabei die Funktion der Gelenkkugel.

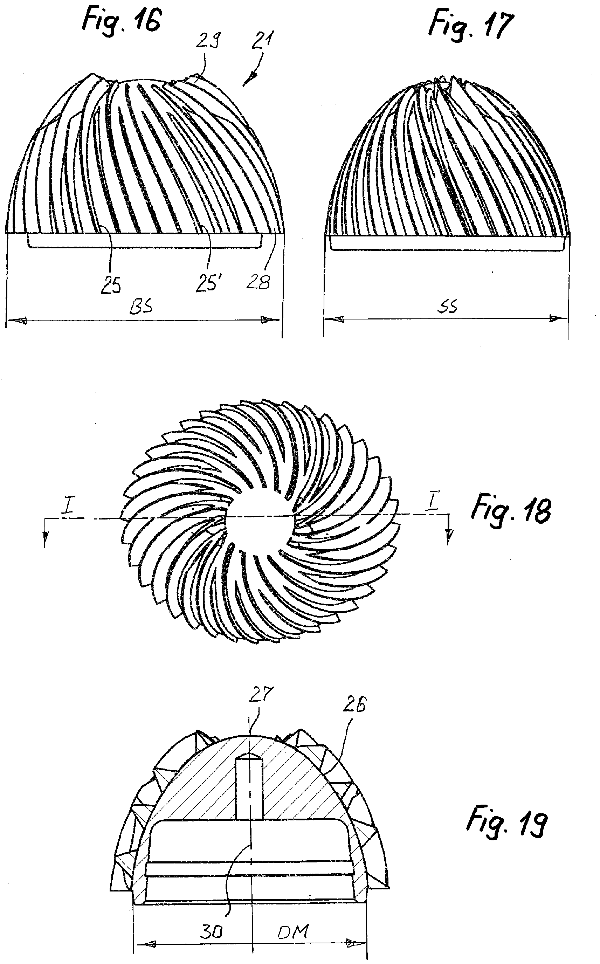

In den Figuren 16 bis 19 sind weitere Details eines Humerusankers 21 dargestellt. Augenfällig ist dabei die im Vergleich zur Hüftgelenkpfanne unterschiedliche Querschnitts- und Grundrissform. Wie insbesondere aus Figur 19 ersichtlich ist, hat die Aussenmantelfläche 26 eine im Querschnitt ellipsoide Konfiguration mit einer rippenfreien aber geschlossenen Polfläche 27. Die einzelnen Rippen 25, 25' erstrecken sich wechselweise bis zur Polfläche oder bis etwas davon entfernt. Trotz des ellipsoiden Querschnitts bezogen auf die Längsmittelachse hat die Schale am Äquator eine kreisrunde Schalenform. Bedingt durch den Schalenquerschnitt und die unterschiedliche Rippenhöhe am Äquator der einzelnen Rippen 25, 25' ergibt sich jedoch auch ein ellipsoider Aussengrundriss mit einer Breitseite BS und mit einer Schmalseite SS. Wie dargestellt verlaufen die einzelnen Rippen beim Humerusanker vorzugsweise ununterbrochen, jedoch teilweise mit sich verändernder Rippenhöhe und unterschiedlicher Rippenlänge. Auch eine Unterbrechung einzelner oder aller Rippen zur Bildung einzelner Zähne wäre jedoch denkbar. Auf der Innenseite des Humerusankers 21 sind geeignete Befestigungsmittel 30 vorgesehen (Figur 19) um den Humeruskopf nach dem Einschlagen zu befestigen.

Analog zur vorstehend beschriebenen Hüftgelenkschale wird nachstehend ebenfalls beispielshaft eine Tabelle mit den Beziehungen zwischen Rippenabstand, Rippenhöhe und insbesondere mit dem Verhältnis zwischen der kumulierten Flankenprojektionsfläche zur Aussenmantelfläche dargestellt. Die Schalengrössen sind dabei für einen Humerusanker naturgemäss kleiner als bei einer Hüftgelenkschale.

| | | | |
|---|---|---|---|
| | **min.** | **Ideal** | **max.** |
| Rippenabstand a | 0.2 | 3 | 4 |
| Rippenhöhe d | 0.1 | 1.1 | 4 |

| **Parameter an Schale Grösse 28 gerechnet Aussenmantelfäche: 1321 mm2** | | | |
|---|---|---|---|
| kum. Flankenprojektionsfläche | 517 | 426 | 1'379 |
| Anzahl Rippen | 456 | 32 | 24 |
| Verhältnis | 0,391 | 0,32 | 1,04 |

| **Parameter an Schale Grösse 38 gerechnet Aussenmantelfäche: 2389 mm2** | | | |
|---|---|---|---|
| kum. Flankenprojektionsfläche | 935 | 739 | 2'337 |
| Anzahl Rippen | 614 | 42 | 32 |
| Verhältnis | 0,391 | 0,31 | 0,98 |

| **Parameter an Schale Grösse 47 gerechnet Aussenmantelfäche: 3619 mm2** | | | |
|---|---|---|---|
| kum. Flankenprojektions-fläche | 1'411 | 1'115 | 3'304 |
| Anzahl Rippen | 754 | 52 | 38 |
| Verhältnis | 0,390 | 0,31 | 0,91 |

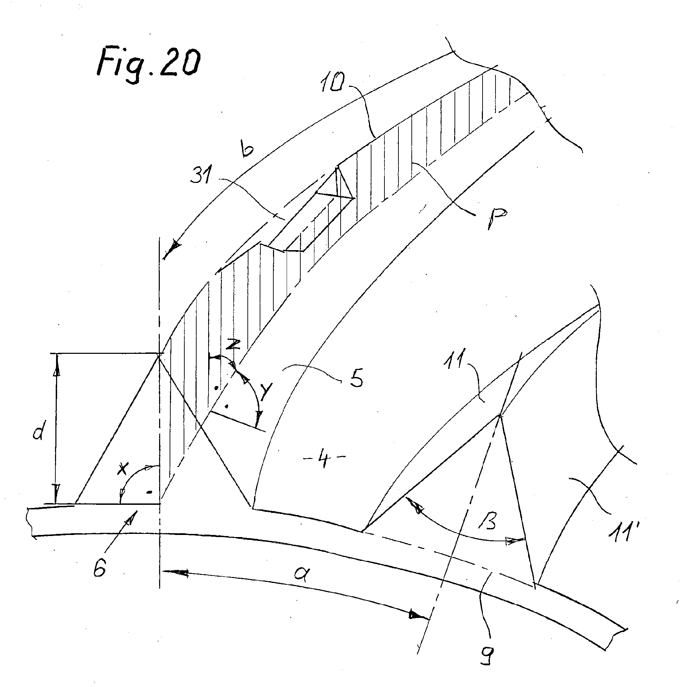

Figur 20 zeigt nochmals schematisch und stark vergrössert die Flankenprojektionsfläche P am Beispiel einer Rippe 5 mit einer Unterbrechung 31 im Rippenscheitel 10. Diese Unterbrechung reicht jedoch nicht bis zum Rippengrund 9, womit sich ersichtlicherweise keine Unterbrechung, sondern lediglich ein Ausschnitt in der Flankenprojektionsfläche P ergibt. Die Rippenlänge b entspricht hier auch der Längserstreckung der Rippe 5 vom äquatorialen Ende 6 bis zum hier nicht dargestellten polseitigen Ende.

Die Flankenprojektionsfläche P verläuft senkrecht zur Aussenmantelfläche 4, wie mit dem Pfeil x angedeutet. Die Rippenflanken 11 und 11' bilden hier einen keilförmigen Querschnitt und zwar bezogen auf eine senkrecht zur Längserstreckung der Rippen und senkrecht zur Aussenmantelfläche verlaufenden Ebene, wie mit den Pfeilen y und z angedeutet. Daraus ergibt sich hier der Flankenwinkel β. Nochmals dargestellt ist hier auch die Rippenhöhe d und der Rippenabstand a als Bogenmass zwischen den Zentren benachbarter Rippen am äquatorseitigen Rippengrund.

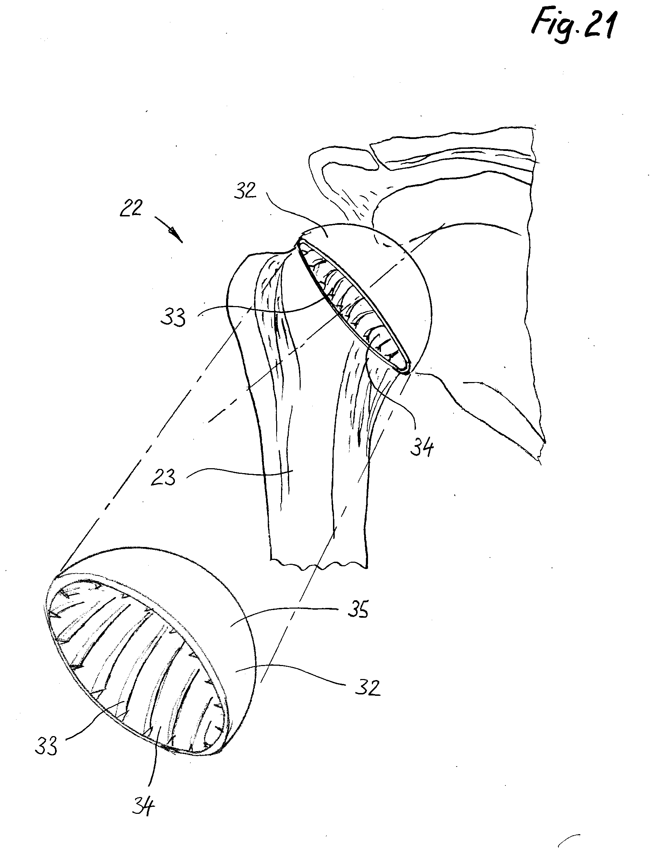

Figur 21 zeigt das Beispiel einer erfindungsgemässen Schale, bei welcher die Rippen nicht an der Aussenmantelfläche, sondern an einer Innenmantelfläche angeordnet sind. Das Beispiel betrifft eine schaftfreie Schultergelenkprothese 22, wobei eine halbkugelförmige Ankerschale 32 direkt den Oberflächenersatz am Humerus 23 bildet. Im Gegensatz zur Schultergelenkprothese gemäss Figur 15 ist ein kein Humerusanker erforderlich, weil die Ankerschale 32 direkt mit den Innenrippen 33 auf den Humerus aufgeschlagen und auf diese Weise verankert wird. Die Innenrippen 33 verlaufen dabei auf der Innenmantelfläche 34 auf die gleiche Art und Weise gegen den Pol hin, wie wenn die Rippen auf einer Aussenmantelfläche angeordnet wären. Beim vorliegenden Ausführungsbeispiel bildet die Aussenmantelfläche 35 der Ankerschale 32 unmittelbar die Gelenkfläche, analog zu Humeruskopf 24 beim Ausführungsbeispiel gemäss Figur 15. Ersichtlicherweise erfolgt beim Aufschlagen der Ankerschale 32 auf den Humerus 23 ebenfalls eine Drehung um wenige Winkelgrade bis zum Erreichen der Endposition.

Selbstverständlich könnte eine erfindungsgemässe Schale auch noch für alternative Gelenkkonstruktionen eingesetzt werden, so beispielsweise für eine inverse Schulterprothese, bei welcher die Gelenkpfanne am Schulterblatt durch eine Kunstgelenkkugel ersetzt wird und die Gelenkkugel am Oberarm durch eine Kunstgelenkpfanne. Auch in einem derartigen Fall könnte die Gelenkpfanne schaftlos im Humerus verankert werden.

## Patentansprüche

1. Schale (1, 21, 32) zum Einschlagen in eine Knochensubstanz für eine Gelenkprothese (2, 22) mit einer im Querschnitt konvex gekrümmten, insbesondere sphärischen Aussenmantelfläche (4, 26), oder mit einer konvex gekrümmten, insbesondere sphärischen Innenmantelfläche (34), auf der eine Mehrzahl von Rippen (5, 25) angeordnet sind, wobei sich alle Rippen gleichsinnig unter einem vorzugsweise zunehmenden Steigungswinkel (α) von 45° bis 85° am äquatorialen Ende (6) zu einem polseitigen Ende (7) auf der Aussenmantelfläche bzw. auf der Innenmantelfläche erstrecken, **dadurch gekennzeichnet, dass** jede Rippe eine Flankenprojektionsfläche (P) aufweist, die sich auf eine durch die Längserstreckung der Rippe und senkrecht zur Aussenmantelfläche bzw. zur Innenmantelfläche verlaufende Ebene bezieht, wobei die Summe der Flankenprojektionsfläche aller Rippen wenigstens einem Fünftel der ganzen Aussenmantelfläche bzw. der ganzen Innenmantelfläche entspricht.

2. Schale nach Anspruch 1, wobei das Verhältnis zwischen der Summe der Flankenprojektionsfläche aller Rippen und der Aussenmantelfläche bzw. der Innenmantelfläche im Bereich von 0,2:1 bis 1:1 liegt.

3. Schale nach Anspruch 1 oder 2, wobei auf der Aussenmantelfläche bzw. auf der Innenmantelfläche wenigstens 20 und vorzugsweise zwischen 30 und 80 Rippen angeordnet sind.

4. Schale nach einem der Ansprüche 1 bis 3, wobei der Abstand (a) zwischen benachbarten Rippen am äquatorialen Ende (6) von Rippenmitte zu Rippenmitte auf der Aussenmantelfläche oder auf der Innenmantelfläche im Bereich von 0,2 mm bis 4 mm liegt.

5. Schale nach einem der Ansprüche 1 bis 4, wobei sich wenigstens 30%, vorzugsweise wenigstens 50% der Rippen (5) über mehr als die Hälfte der Höhe (c) der Schale, bezogen auf deren Längsmittelachse (8), erstrecken.

6. Schale nach einem der Ansprüche 1 bis 5, wobei auf der Aussenmantelfläche (4) oder auf der Innenmantelfläche Rippen (5) unterschiedlicher Länge, vorzugsweise in regelmässiger Abfolge, angeordnet sind.

7. Schale nach einem der Ansprüche 1 bis 6, wobei die Rippenhöhe (d) vom Rippengrund (9) zum Rippenscheitel (10) im Bereich von 0,1 mm bis 4 mm liegt.

8. Schale nach einem der Ansprüche 1 bis 7, wobei auf der Aussenmantelfläche (4) oder auf der Innenmantelfläche Rippen (5) unterschiedlicher Rippenhöhe (d), vorzugsweise in regelmässiger Abfolge angeordnet sind.

9. Schale nach einem der Ansprüche 1 bis 8, wobei bezogen auf den Umfang der Aussenmantelfläche oder der Innenmantelfläche auf einem bestimmten Breitengrad Rippen unterschiedlicher Rippenhöhe (d) angeordnet sind.

10. Schale nach einem der Ansprüche 1 bis 9, wobei die Rippen (5) einen sich vom Rippengrund (9) zum Rippenscheitel (10) verjüngenden Querschnitt, bezogen auf eine senkrecht zur Längserstreckung der Rippe und senkrecht zur Aussenmantelfläche bzw. zur Innenmantelfläche verlaufende Ebene vorzugsweise einen keilförmigen Querschnitt, aufweisen.

11. Schale nach einem der Ansprüche 1 bis 10, wobei die gegenseitigen Flankenflächen der einzelnen Rippen (5) unterschiedliche Rauheitswerte aufweisen, derart, dass die beim Einschlagen höher belasteten Flanken den geringeren Rauheitswert aufweisen als die beim Ausziehen höher belasteten Flanken.

12. Schale nach einem der Ansprüche 1 bis 11, wobei wenigstens ein Teil der Rippen (5) zur Bildung einzelner Rippenzähne (12) unterbrochen sind.

13. Schale nach Anspruch 12, wobei die Rippenzähne (12) einer Rippe (5) zwischen dem äquatorialen Ende (6) und dem polseitigen Ende (7) eine unterschiedliche Konfiguration aufweisen.

14. Schale nach Anspruch 12 oder 13, wobei die Rippenzähne (12) vorzugsweise unregelmässige, mehrflächige Körper bilden, mit einem viereckigen Grundriss, zwei gegeneinander geneigten Flankenseiten (15, 15'), einer dem Pol zugewandten Polseite (16) und einer dem Äquator zugewandten Äquatorseite (17) .

15. Schale nach Anspruch 13 oder 14, wobei die aufeinanderfolgenden Rippenzähne einer Rippe derart konfiguriert sind, dass in Einschlagrichtung (e) eine Verdrängung von Knochenmaterial und in Ausdrehrichtung (f) eine Verankerung im Knochenmaterial stattfindet.

16. Schale nach einem der Ansprüche 12 bis 15, wobei sich die Zwischenräume (13) zwischen den Rippenzähnen (12) nicht bis zur Aussenmantelfläche (4) bzw. zur Innenmantelfläche erstrecken.

17. Schale nach einem der Ansprüche 1 bis 16, wobei die Aussenmantelfläche (4) bzw. die Innenmantelfläche und/oder die Rippen (5) ganz oder teilweise mit einer osteoinduktiven Beschichtung (14) versehen sind.

18. Schale nach einem der Ansprüche 1 bis 17, wobei die Schale eine Gelenkschale (1) für eine Hüftgelenkprothese (2) zum Einschlagen in ein Acetabulum ist, wobei das Verhältnis zwischen der Summe der Flankenprojektionsfläche aller Rippen und der Aussenmantelfläche im Bereich von 0,2:1 bis 0,8:1 liegt.

19. Schale nach einem der Ansprüche 1 bis 17, wobei die Schale ein Humerusanker (21) zum Einschlagen in einen Humerus (23) ist, wobei das Verhältnis zwischen der Summe der Flankenprojektionsfläche aller Rippen und der Aussenmantelfläche im Bereich von 0,3:1 bis 1:1 liegt.

20. Schale nach einem der Ansprüche 1 bis 19, wobei der Verlauf der Rippen so gewählt ist, dass jede Drehung um einen vorbestimmten Winkelwert einen konstanten Vortrieb der Schale bewirkt.

## Claims

1. A shell (1, 21, 32) to be driven into a bone substance for a prosthetic joint (2, 22), having an outer lateral surface (4, 26) which is convexly curved, especially spherical, in cross-section, or having an inner lateral surface (34) which is convexly curved, especially spherical, on which outer or inner lateral surface there is arranged a plurality of ribs (5, 25), wherein all ribs extend in the same direction under a preferably increasing pitch angle (α) from 45° to 85° at the equatorial end (6) toward a pole-sided end (7) on the outer lateral surface or on the inner lateral surface, **characterised in that** each rib has a flank projection area (P) based on a plane running through the longitudinal extent of the rib and perpendicularly to the outer lateral surface or to the inner lateral surface, wherein the sum of the flank projection area of all ribs corresponds to at least one fifth of the entire outer lateral surface or the entire inner lateral surface.

2. The shell as claimed in claim 1, wherein the ratio between the sum of the flank projection area of all ribs and the outer lateral surface or the inner lateral surface is in the range from 0.2:1 to 1:1.

3. The shell as claimed in claim 1 or 2, wherein at least 20 and preferably between 30 and 80 ribs are arranged on the outer lateral surface or on the inner lateral surface.

4. The shell as claimed in any of claims 1 to 3, wherein the interval (a) between adjacent ribs at the equatorial end (6) from rib center to rib center on the outer lateral surface or on the inner lateral surface is in the range from 0.2 mm to 4 mm.

5. The shell as claimed in any of claims 1 to 4, wherein at least 30%, preferably at least 50%, of the ribs (5) extend over more than half the height (c) of the shell, based on the longitudinal central axis (8) thereof.

6. The shell as claimed in any of claims 1 to 5, wherein ribs (5) of differing length are arranged, preferably in a regular sequence, on the outer lateral surface (4) or on the inner lateral surface.

7. The shell as claimed in any of claims 1 to 6, wherein the rib height (d) from the rib foundation (9) to the rib vertex (10) is in the range from 0.1 mm to 4 mm.

8. The shell as claimed in any of claims 1 to 7, wherein ribs (5) of differing rib height (d) are arranged, preferably in a regular sequence, on the outer lateral surface (4) or on the inner lateral surface.

9. The shell as claimed in any of claims 1 to 8, wherein ribs of differing rib height (d) are arranged on a certain latitude based on the circumference of the outer lateral surface or the inner lateral surface.

10. The shell as claimed in any of claims 1 to 9, wherein the ribs (5) have a cross-section which tapers from the rib foundation (9) toward the rib vertex (10), based on a plane running perpendicularly to the longitudinal extent of the rib and perpendicularly to the outer lateral surface or to the inner lateral surface, preferably a wedge-shaped cross-section.

11. The shell as claimed in any of claims 1 to 10, wherein the opposing flank surfaces (20, 20') of the individual ribs (5) have different roughness values, such that the flanks subjected to a greater load during drive-in have the lower roughness value than the flanks subjected to a greater load during pull-out.

12. The shell as claimed in any of claims 1 to 11, wherein at least some of the ribs (5) are interrupted to form individual rib teeth (12).

13. The shell as claimed in claim 12, wherein the rib teeth (12) of a rib (5) have a differing configuration between the equatorial end (6) and the pole-sided end (7).

14. The shell as claimed in claim 12 or 13, wherein the rib teeth (12) preferably form irregular, multisurface bodies having a quadrangular footprint, two flank sides (15, 15') inclined against one another, a pole side (16) facing the pole, and an equator side (17) facing the equator.

15. The shell as claimed in claim 13 or 14, wherein the successive rib teeth of a rib are configured such that a displacement of bone material takes place in the drive-in direction (e) and an anchoring in the bone material takes place in the screw-out direction (f) .

16. The shell as claimed in any of claims 12 to 15, wherein the gaps (13) between the rib teeth (12) do not extend as far as the outer lateral surface (4) or the inner lateral surface.

17. The shell as claimed in any of claims 1 to 16, wherein the outer lateral surface (4) or the inner lateral surface and/or the ribs (5) are provided with an osteoinductive coating (14) in full or in part.

18. The shell as claimed in any of claims 1 to 17, wherein the shell is a joint shell (1) for a prosthetic hip joint (2) to be driven into an acetabulum, wherein the ratio between the sum of the flank projection area of all ribs and the outer lateral surface is in the range from 0.2:1 to 0.8:1.

19. The shell as claimed in any of claims 1 to 17, wherein the shell is a humeral anchor (21) to be driven into a humerus (23), wherein the ratio between the sum of the flank projection area of all ribs and the outer lateral surface is in the range from 0.3:1 to 1:1.

20. The shell as claimed in any of claims 1 to 19, wherein the course of the ribs is chosen such that each rotation by a predetermined angular value brings about a constant advance of the shell.

## Revendications

1. Coque (1, 21, 32) destinée à être enfoncée dans une substance osseuse pour une prothèse articulaire (2, 22), comprenant une surface d'enveloppe extérieure (4, 26) de courbure convexe en section transversale, en particulier sphérique, ou comprenant une surface d'enveloppe intérieure (34) de courbure convexe, en particulier sphérique, sur laquelle sont disposées une pluralité de nervures (5, 25),
toutes les nervures s'étendant dans le même sens suivant un angle de pente (α) de préférence croissant, de 45° à 85° à l'extrémité équatoriale (6) jusqu'à une extrémité polaire (7) sur la surface d'enveloppe extérieure ou sur la surface d'enveloppe intérieure,
**caractérisée en ce que** chaque nervure présente une surface en projection sur un flanc (P) qui se rapporte à un plan s'étendant à travers l'étendue longitudinale de la nervure et perpendiculairement à la surface d'enveloppe extérieure ou à la surface d'enveloppe intérieure, la somme de la surface en projection sur un flanc de toutes les nervures correspondant à au moins un cinquième de toute la surface d'enveloppe extérieure ou de toute la surface d'enveloppe intérieure.

2. Coque selon la revendication 1, dans laquelle le rapport entre la somme de la surface en projection sur un flanc de toutes les nervures et la surface d'enveloppe extérieure ou la surface d'enveloppe intérieure est de l'ordre de 0,2:1 à 1:1.

3. Coque selon la revendication 1 ou 2, dans laquelle au moins 20 et de préférence entre 30 et 80 nervures sont disposées sur la surface d'enveloppe extérieure ou sur la surface d'enveloppe intérieure.

4. Coque selon l'une quelconque des revendications 1 à 3, dans laquelle la distance (a) entre des nervures adjacentes au niveau de l'extrémité équatoriale (6) d'un centre de nervure à un autre sur la surface d'enveloppe extérieure ou sur la surface d'enveloppe intérieure est de l'ordre de 0,2 mm à 4 mm.

5. Coque selon l'une quelconque des revendications 1 à 4, dans laquelle au moins 30 %, de préférence au moins 50 % des nervures (5) s'étendent sur plus de la moitié de la hauteur (c) de la coque, par rapport à son axe médian longitudinal (8).

6. Coque selon l'une quelconque des revendications 1 à 5, dans laquelle des nervures (5) de longueurs différentes sont disposées de préférence en succession régulière sur la surface d'enveloppe extérieure (4) ou sur la surface d'enveloppe intérieure.

7. Coque selon l'une quelconque des revendications 1 à 6, dans laquelle la hauteur des nervures (d) de la base de nervure (9) au sommet de nervure (10) est de l'ordre de 0,1 mm à 4 mm.

8. Coque selon l'une quelconque des revendications 1 à 7, dans laquelle des nervures (5) de hauteurs de nervure (d) différentes sont disposées de préférence en succession régulière sur la surface d'enveloppe extérieure (4) ou sur la surface d'enveloppe intérieure.

9. Coque selon l'une quelconque des revendications 1 à 8, dans laquelle des nervures de hauteurs de nervure différentes (d) sont disposées à une certaine latitude, par rapport à la périphérie de la surface d'enveloppe extérieure ou de la surface d'enveloppe intérieure.

10. Coque selon l'une quelconque des revendications 1 à 9, dans laquelle les nervures (5) présentent une section transversale se rétrécissant depuis le fond de rainure (9) jusqu'au sommet de rainure (10), par rapport à un plan s'étendant perpendiculairement à l'étendue longitudinale de la nervure et perpendiculairement à la surface d'enveloppe extérieure ou à la surface d'enveloppe intérieure, de préférence une section transversale en forme de cale.

11. Coque selon l'une quelconque des revendications 1 à 10, dans laquelle les surfaces de flanc opposées des nervures individuelles (5) présentent des valeurs de rugosité différentes de telle sorte que les flancs plus fortement sollicités lors de l'enfoncement présentent une plus faible valeur de rugosité que les flancs plus fortement sollicités lors du retrait.

12. Coque selon l'une quelconque des revendications 1 à 11, dans laquelle au moins une partie des nervures (5) sont interrompues pour former des dents de nervures individuelles (12).

13. Coque selon la revendication 12, dans laquelle les dents de nervure (12) d'une nervure (5) présentent une configuration différente, entre l'extrémité équatoriale (6) et l'extrémité polaire (7).

14. Coque selon la revendication 12 ou 13, dans laquelle les dents de nervure (12) forment des corps à plusieurs surfaces, de préférence irréguliers, avec une projection horizontale quadrangulaire, deux côtés de flanc inclinés l'un vers l'autre (15, 15'), un côté polaire (16) tourné vers le pôle et un côté équatorial (17) tourné vers l'équateur.

15. Coque selon la revendication 13 ou 14, dans laquelle les dents de nervures successives d'une nervure sont configurées de telle sorte qu'il se produise dans la direction d'enfoncement (e), un refoulement de la matière osseuse et dans la direction de retrait (f), un ancrage dans la matière osseuse.

16. Coque selon l'une quelconque des revendications 12 à 15, dans laquelle les espaces intermédiaires (13) entre les dents de nervure (12) ne s'étendent pas jusqu'à la surface d'enveloppe extérieure (4) ou jusqu'à la surface d'enveloppe intérieure.

17. Coque selon l'une quelconque des revendications 1 à 16, dans laquelle la surface d'enveloppe extérieure (4) ou la surface d'enveloppe intérieure et/ou les nervures (5) sont pourvues en partie ou complètement d'un revêtement ostéoinductif (14).

18. Coque selon l'une quelconque des revendications 1 à 17, la coque étant une cupule acétabulaire (1) pour une prothèse d'articulation de la hanche (2) destinée à être enfoncée dans une cavité cotyloïdienne, le rapport entre la somme de la surface en projection sur un flanc de toutes les nervures et la surface d'enveloppe extérieure étant de l'ordre de 0,2:1 à 0,8:1.

19. Coque selon l'une quelconque des revendications 1 à 17, la coque étant un ancrage de l'humérus (21) destiné à être enfoncé dans un humérus (23), le rapport entre la somme de la surface en projection sur un flanc de toutes les nervures et la surface d'enveloppe extérieure étant de l'ordre de 0,3:1 à 1:1.

20. Coque selon l'une quelconque des revendications 1 à 19, dans laquelle l'allure des nervures est choisie de telle sorte que chaque rotation autour d'une valeur angulaire prédéterminée provoque une avance constante de la coque.
